# EUROPÄISCHE PATENTANMELDUNG

(11) **EP 0 575 833 A2**
(43) Veröffentlichungstag der Anmeldung: **29.12.1993**
(21) Anmeldenummer: 93109355.3
(22) Anmeldetag: 11.06.1993
(51) Int. Cl.: A61B 1/06, A61B 1/04

(54) **Vorrichtung zur Lichtversorgung von Endoskopen**

(30) Priorität: 24.06.1992 DE 4220633
(71) Anmelder: Richard Wolf GmbH, D-75438 Knittlingen (DE)
(72) Erfinder: Häfele, Ulrich, W-7137 Sternenfels-Diefenbach (DE); Vögele, Michael, W-7539 Kämpfelbach-Ersingen (DE)
(74) Vertreter: Wilcken, Thomas, Dipl.-Ing.

(57) **Zusammenfassung**

Die Vorrichtung weist ein Lichtquellengerät (6) auf, das wahlweise weißes Dauerlicht oder mittels einer mehrere Farbflächenbereiche aufweisenden Filtereinrichtung (15) mit zwei drehantreibbaren Filterscheiben (26, 27) sequentielles Farblicht in das jeweilige Endoskop einspeist. Zur Erzielung eines vereinfachten und baulich kompakten Lichtquellengerätes (6) ist die eine Filterscheibe (26) mit den Farbsegmenten rot (R), grün (G) und weiß (W) und die andere Filterscheibe (27) mit den Farbsegmenten blau (B) und weiß (W) versehen, wobei beide Filterscheiben (26, 27) auf einer gemeinsamen Drehachse (28) rotierbar und relativ zueinander einstellbar sowie derart fixierbar angeordnet sind, daß in einer ersten Arbeitsstellung der Filterscheiben das weiße Farbsegment (W) der einen Filterscheibe (26) mit dem blauen Farbsegment (B) der anderen Filterscheibe (27) in Überdeckung und in einer zweiten Arbeitsstellung der Filterscheiben das weiße Farbsegment (W) der einen Filterscheibe (26) mit dem weißen Farbsegment (W) der anderen Filterscheibe (27) achsparallel ausgerichtet ist.

## Beschreibung

Die Erfindung geht aus von einer Vorrichtung zur Lichtversorgung von Endoskopen, mit einem Lichtquellengerät, das wahlweise weißes Dauerlicht oder mittels einer mehrere Farbflächenbereiche aufweisenden Filtereinrichtung mit zwei drehantreibbaren Filterscheiben sequentielles Farblicht in das jeweilige Endoskop einspeist.

Eine derartige Vorrichtung ist in der US-Patentschrift 4 951 133 beschrieben. Diese Vorrichtung weist eine Filtereinrichtung auf, die von einem Lichtquellengerät mit Licht durchstrahlt wird, um weißes Dauerlicht oder sequentielles Farblicht in den Lichtleiter am proximalen Ende eines Endoskopes einzuspeisen. Die Filtereinrichtung hat eine erste, umlaufende Filterscheibe mit den drei Farbsegmenten rot, grün und blau und eine zweite, drehverstellbare Scheibe mit einem Infrarotstrahlung unterdrückenden Filterteil und einem freien Durchgang für das weiße Licht des Lichtquellengerätes. Zur Einleitung von weißem Dauerlicht in den Lichtleiter des Endoskops wird die zweite Scheibe mit ihrem freien Durchgang in den Strahlengang der Lichtquelle verstellt, während die erste Filterscheibe aus dem Strahlengang der Lichtquelle seitlich herausgefahren wird. Soll sequentielles Farblicht in den Lichtleiter eingespeist werden, wird die zweite Scheibe auf ihr Filterteil für die Infrarotunterdrückung eingestellt und die erste Filterscheibe wieder in den Strahlengang der Lichtquelle zurückgestellt. Die erste Filterscheibe benötigt außer ihrem Drehantrieb noch einen gesonderten Antrieb für die eben erläuterte seitliche Verfahrbarkeit. Diese Bauweise für die Filtereinrichtung ist material- und kostenaufwendig und erfordert einen erhöhten Platzbedarf.

Die Aufgabe der Erfindung besteht in der Verbesserung der einleitend angeführten Vorrichtung dahingehend, daß zum einen mit einer vereinfachten, kostengünstigen und kompakten Konstruktion des Lichtquellengerätes wahlweise weißes Dauerlicht oder sequentielles Farblicht als Beleuchtungslicht für verschiedenartige Endoskope zur Verfügung gestellt werden kann und daß die Vorrichtung zum anderen als Einschub verwendbar ist..

Die Lösung der Aufgabe ist in dem Patentanspruch 1 angegeben.

Durch diese Lösung entfallen zwei Antriebsmotoren für die Filtereinrichtung, weil die beiden Filterscheiben auf einer gemeinsamen Antriebswelle sitzen, die von nur einem einzigen Motor angetrieben wird, und weil ein seitliches Herausfahren von Filterscheiben nicht mehr erforderlich ist. Insgesamt wird dadurch ebenfalls eine kompakte Bauweise des Lichtquellengerätes und damit auch der erfindungsgemäßen Vorrichtung erreicht. Infolgedessen eignet sich die Vorrichtung besonders gut als Baueinheit in Form eines sogenannten Einschubes.

Eine bevorzugte Ausgestaltung der erfindungsgemäßen Vorrichtung besteht darin, daß die eine Filterscheibe starr auf einer Antriebswelle und die andere Filterscheibe frei drehbar auf dieser Antriebswelle angeordnet ist und daß beide Filterscheiben mittels einer Federeinrichtung miteinander gekoppelt sind. Dabei kann die eine Filterscheibe zwei voneinander beabstandete Anschläge und die andere Filterscheibe einen mit den vorgenannten Anschlägen zusammenwirkenden Anschlag zum Einstellen der beiden Arbeitsstellungen der Filterscheiben aufweisen. Vorzugsweise ist den Filterscheiben eine Fixiereinrichtung zum Feststellen ihrer Arbeitsstellungen zugeordnet, wobei die Fixiereinrichtung aus einem Rastmechanismus für jede in diesem Fall mit einer Rastkerbe versehenen Filterscheibe besteht. Weiterhin ist es vorteilhaft, daß das weiße Farbsegment der einen Filterscheibe einen Winkelbereich von 120 ° umfaßt, während das weiße Farbsegment der anderen Filterscheibe einen Winkelbereich von 240 ° umfaßt. Insgesamt ist diese Ausgestaltung der Filtereinrichtung funktionssicher, sehr einfach aufgebaut und sehr kostengünstig herzustellen.

Die Erfindung ist nachstehend anhand eines in den anliegenden Zeichnungen dargestellten Ausführungsbeispieles näher erläutert. Es zeigen:
- Figur 1: die Lichtversorgung verschiedener Endoskope von einem einzigen Lichtquellengerät,
- Figur 2: den vereinfachten prinzipiellen Aufbau des Lichtquellengerätes,
- Figuren 3 und 4: die Stromkurven für die Stromversorgung des Lichtquellengerätes,
- Figur 5: eine erste Ausführungsform einer Filtereinrichtung des Lichtquellengerätes,
- Figur 6: eine zweite Ausführungsform einer Filtereinrichtung des Lichtquellengerätes.

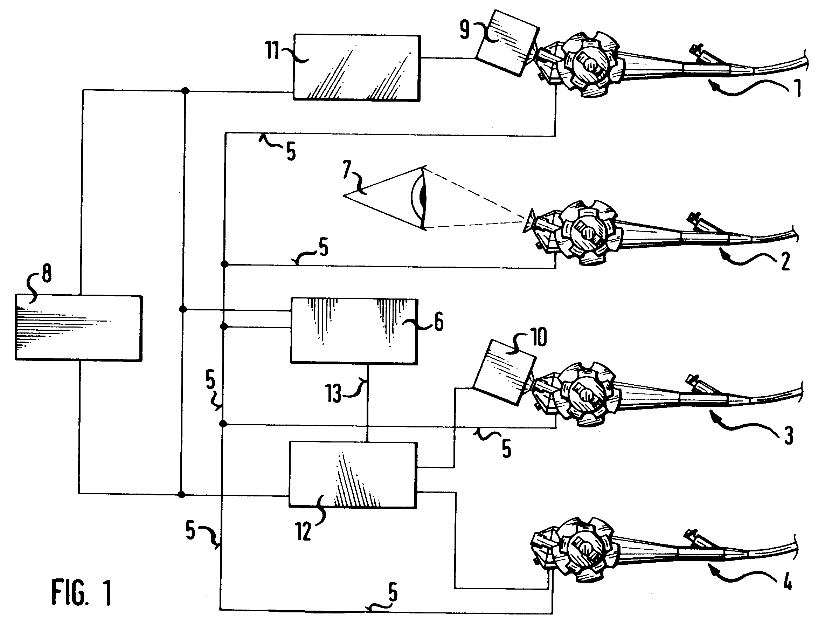

In Figur 1 sind mehrere Beispiele von verschiedenen Endoskopen 1,2,3 und 4 dargestellt, die über Lichtleiterkabel 5 an ein einziges Lichtquellengerät 6 angeschlossen werden können, um Beleuchtungslicht aus dem Gerät 6 für das jeweilige Endoskop zur Verfügung zu stellen. Die dargestellten Endoskope sind flexible Endoskope, z.B. Fiberskope, es können jedoch auch starre Endoskope sein. Während mit dem Endoskop 2 z. B. eine Körperhöhle über das Endoskopokular mit bloßem Auge 7 betrachtet wird, erfolgt die Betrachtung mit anderen Endoskopen mittels eines Monitors 8 und zwischengeschalteter Festkörperbildwandler. Die Endoskope 1 und 3 weisen je einen an ihrem Okular ansetzbaren Festkörperbildwandler 9 bzw. 10 auf, der in Verbindung mit einer Kamera 11 bzw. einem Viedoskop-Prozessor 12 verwendet wird, um das gewünschte Bild über entsprechende Videosignal-Leitungen auf dem Monitor 8 erscheinen zu lassen, wie es aus Figur 1 zu erkennen ist. Bei dem Endoskop 4 ist der Wandler in das distale Ende des Endoskopes eingegliedert (nicht gezeigt).

Dem Endoskop 1 ist die Kamera 11 mit dem Farb-CCD-Bildwandler-Element zugeordnet. Für das Endoskop 1 und auch für das Endoskop 2 ist weißes Dauerlicht von dem Lichtquellengerät 6 erforderlich.

An das Endoskop 3 ist das Schwarz/Weiß(S/W)-CCD-Bildwandler-Element 10 angeschlossen. Daher muß dieses Endoskop mit sequentiellem Farblicht versorgt werden, um auf den Monitor 8 ein Farbbild erzeugen zu können. Bei dem Endoskop 4 handelt es sich um ein Videoendoskop, das sowohl mit sequentiellem Farblicht als auch mit weißem Dauerlicht versorgt wird. Das sequentielle Farblicht ist erforderlich, wenn das Videoendoskop mit einem S/W-CCD-Bildwandler-Element versehen ist; und es ist weißes Dauerlicht erforderlich, wenn dieses Endoskop mit einem Farb-CCD-Bildwandler-Element versehen ist. Die beiden Endoskope 3 und 4 sind des weiteren an den Videoskop-Prozessor angeschlossen, der wiederum über eine Steuerleitungseinrichtung 13 an das Lichtquellengerät 6 angeschlossen ist, um die Lichtversorgung der beiden Endoskope 3 und 4 bestimmungsgemäß steuern zu können.

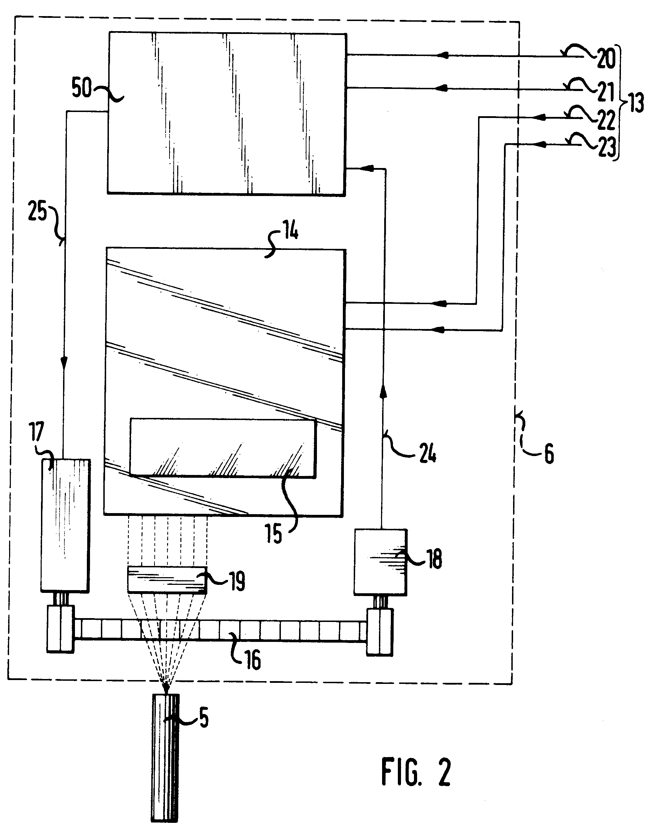

Figur 2 zeigt schematisch den Grundaufbau und die Steuerung des Lichtquellengerätes 6. Dieses Gerät umfaßt eine Lichterzeugungseinheit 14 mit einer Filtereinrichtung 15, eine Blende 16, einen Motor 17 zum Antrieb der Blende 16, ein starr mit der Blende 16 gekoppeltes Potentiometer 18, eine Fokuslinse 19, die das Licht der Einheit 14 auf das Lichtleiterkabel 5 fokussiert, und eine Regeleinrichtung 50 zum Einstellen der Durchlaßöffnung der Blende 16. Die an das Gerät 6 angeschlossene Steuerleitungseinrichtung 13 umfaßt die einzelnen Steuerleitungen 20,21,22 und 23, die entsprechend an die Blendenregeleinrichtung 50 und an die Lichterzeugungseinheit 14 angeschlossen sind. Das Potentiometer ist über eine Leitung 24 an die Einrichtung 50 angeschlossen, von der eine Leitung 25 zum Motor 17 führt. Aus dem Videosignal der Leitung 20 gewinnt die Regeleinrichtung 50 eine der Helligkeit des betreffenden Videobildes entsprechende Spannung. Aus dieser Spannung wird die Steuerspannung für den Blendenmotor 17 erzeugt. Je nach dem Wert der Steuerspannung wird die Blende 16 geöffnet oder geschlossen, bis die optimale Helligkeit des aus der Einheit 14 austretenden Lichtes erreicht ist. Das starr mit der radförmigen Blende gekoppelte Potentiometer 18 ermittelt die Blendenstellung, die zur Erzeugung der Steuerspannung für den Blendenmotor 17 miteinbezogen wird. Über die Leitung 22 wird die Einheit 14 in der Licht-Betriebsart gesteuert, und zwar ob weißes Dauerlicht oder sequentielles Farblicht gewünscht wird. Über die Synchronisationsleitung 23 wird die Filtereinrichtung 15 gesteuert, um bestimmungsgemäß sequentielles Farblicht fehlerfrei in das Lichtleiterkabel 5 einspeisen zu können, wie noch klar wird.

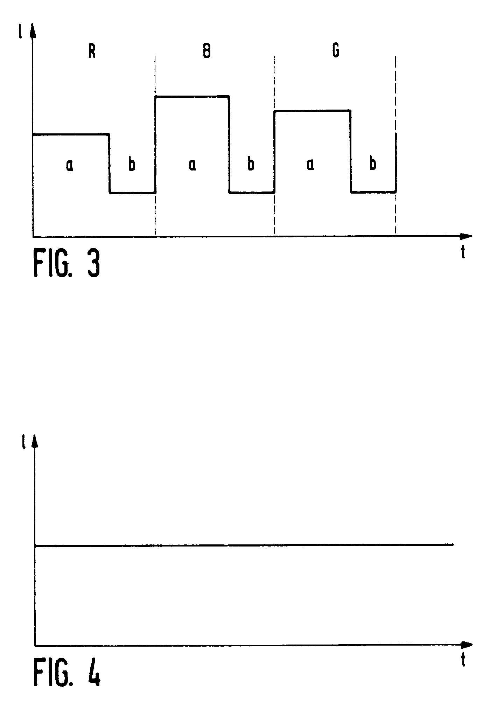

Die Figuren 3 und 4 zeigen die Stromkurven für den Lampenstrom der Lichterzeugungseinheit 14. Man erkennt, daß der Lampenstrom im Falle der Betriebsart "Sequentielles Farblicht" gemäß Fig. 3 einen im wesentlichen rechteckigen Verlauf hat. Während der Zeiten a wird das jeweilige CCD-Element belichtet, während dieses Element während der Zeiten b ausgelesen wird. Während der Auslesephase wird der Lampenstrom also auf ein Minimum reduziert, was zur Folge hat, daß die Lampe der Einheit 14 während der Belichtungsphase mit einem größeren Strom als dem Nennstrom betrieben werden kann. Dies hat einen erhöhten Wirkungsgrad der Lichterzeugungseinheit 14 zur Folge. Gemäß Figur 4 ist der Lampenstrom bei der Betriebsart "Weißes Dauerlicht" auf konstantem Niveau.

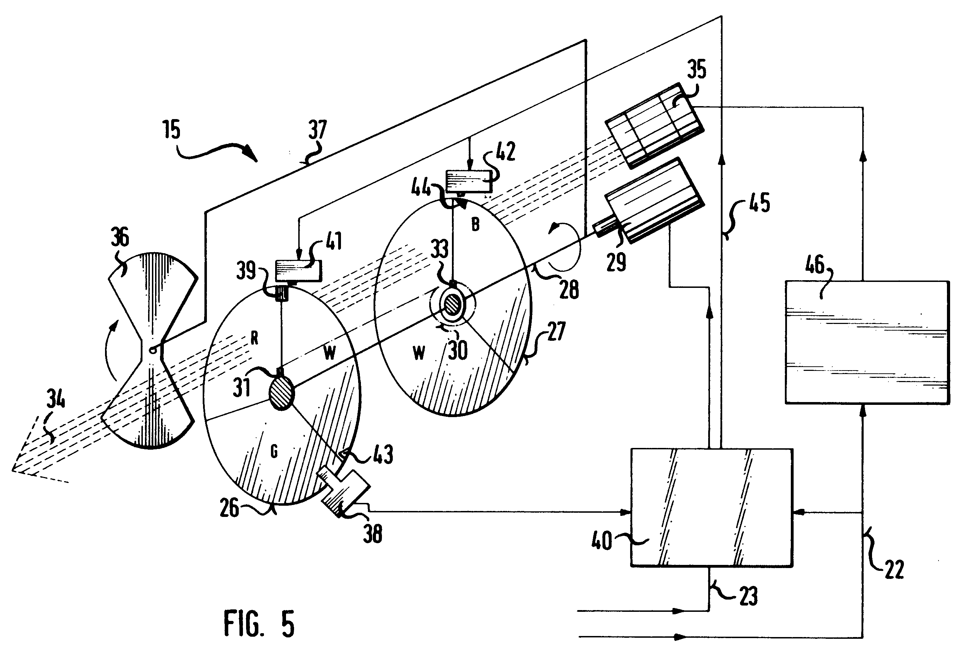

In Figur 5 ist die in Figur 2 schematisch angedeutete Filtereinrichtung 15 in einer ersten Ausführungsform im einzelnen gezeigt. Man erkennt eine erste radförmige Filterscheibe 26 und dahinter eine zweite radförmige Filterscheibe 27, wobei beide Filterscheiben auf einer gemeinsamen Antriebswelle 28 angeordnet sind, die von einem Elektromotor 29 angetrieben wird. Die erste Filterscheibe 26 ist drehfest auf der Welle 28 angeordnet; die zweite Filterscheibe 27 ist frei drehbar auf der Welle 28 angeordnet. Beide Filterscheiben 26,27 sind mittels einer Federeinrichtung 30 gekoppelt, derart, daß die beiden Filterscheiben relativ zueinander drehverstellbar sind. Es ist klar, daß sich beide Scheiben 26,27 synchron drehen, wenn die Welle 28 angetrieben wird. Während die erste Scheibe 26 mit einem Anschlag 31 in ihrem Zentralbereich versehen ist, weist die zweite Scheibe 27 ebenfalls einen Anschlag 33 in ihrem Zentralbereich auf. Der Anschlag 33 arbeitet mit dem Anschla 31 zusammen, um die erste Arbeitsstellung der beiden Filterscheiben zueinander genau festzulegen.

Die erste Filterscheibe 26 ist ferner mit drei gleichen, aneinander angrenzenden Farbsegmenten rot R, grün G und weiß W ausgestattet, wobei diese Farbsegmente einen Winkelbereich von vorzugsweise 120 ° umfassen. Die zweite Filterscheibe 27 ist mit einem weißen Farbsegment W, das vorzugsweise 240 ° umfaßt, und mit einem blauen Farbsegment B, das vorzugsweise einen Winkelbereich von 120 ° umfaßt, ausgestattet. Die Farbsegmente der beiden Filterscheiben liegen sich in der in Figur 5 gezeigten ersten Arbeitsstellung in der Weise direkt axial gegenüber, daß das weiße Farbsegment W der ersten Filterscheibe 26 sich mit dem blauen Farbsegment B der zweiten Filterscheibe in Überdeckung befindet. Da die beiden Filterscheiben üblicherweise im Strahlengang 34 der weißes Licht aussendenden Lampe 35 der Lichterzeugungseinheit 14 liegen, ist es klar, daß bei gemeinsamer Rotation der beiden Filterscheiben in diesem Falle sequentielles Farblicht ausgestrahlt wird.

Wenn es gewünscht wird, kann der ersten Filterscheibe 26 ein rotierendes Flügelrad 36 vorgeordnet sein, um in Zusammenhang mit der Ausstrahlung von sequentiellem Farblicht eine verbesserte Beleuchtungslichtkurve zu erhalten, wie es in Verbindung mit dem DE-Patent 39 08 366 beschrieben ist, worauf hiermit hingewiesen wird. Das Flügelrad 36 wird dabei vorzugsweise über eine Antriebsleitung 37 ebenfalls vom Motor 29 angetrieben.

Ferner ist dem Randbereich der ersten Filterscheibe 26 eine Lichtschranke 38 zugeordnet, wobei die Scheibe 26 an ihrem Rand mit einer Markierung 39 versehen ist. Die Lichtschranke 38 ist wiederum an eine Regeleinrichtung 40 für die Filterscheiben angeschlossen, die auch mit dem Elektromotor 29 zusammenwirkt. Wenn die Filterscheibe 26 rotiert, bewegt sich die Markierung 39 ständig an der Lichtschranke 38 vorbei, so daß diese eine Impulsfolge an die Regeleinrichtung 40 weitergibt. Die Einrichtung 40 vergleicht nun die Impulsfolge der Lichtschranke 38 mit der Impulsfolge des Videoskop-Prozessors 12, die über die Synchronisationsleitung 23 eingeht. Die Regeleinrichtung 40 erhöht oder erniedrigt nun die Steuerspannung für den die Filterscheiben 26,27 antreibenden Motor 29 solange, bis die Synchronisationsimpulsfolge genau mit der Impulsfolge der Lichtschranke übereinstimmt. Daraus resultiert als Ergebnis ein auf den Videoskop-Prozessor 12 synchronisierter Umlauf der Filterscheiben 26,27.

Die in Figur 5 gezeigte Stellung der Filterscheiben 26,27 stellt ihre erste Arbeitsstellung zueinander dar. Diese Stellung ist, wie bereits angedeutet, durch die Federeinrichtung 30, über welche beide Scheiben 26,27 miteinander gekoppelt sind, und durch die Anschläge 31 und 33, die durch die Kraft der Federeinrichtung 30 gegeneinander anliegen, bestimmt. Nur aus Gründen der Klarheit sind die beiden Filterscheiben 26,27 mit größerem Abstand voneinander gezeigt, so daß die Anschläge nicht gegeneinander anliegend gezeigt sind.

Den Filterscheiben 26,27 ist des weiteren eine Fixiereinrichtung zum Feststellen ihrer zweiten Arbeitsstellung zugeordnet. Diese Fixiereinrichtung besteht im hier gezeigten Fall aus je einem Rastmechanismus 41,42 für jede Filterscheibe und aus je einer Rastkerbe 43 und 44 in den Filterscheiben 26 bzw. 27. Die Rastkerbe 43 ist dabei gemäß Figur 5 im unteren Endbereich des weißen Farbsegmentes der Filterscheibe 26 vorgesehen. Die Rastkerbe 44 der anderen Filterscheibe 27 ist gemäß Figur 5 im oberen Bereich des blauen Farbsegmentes der Filterscheibe 27 vorgesehen. Demgemäß sind die beiden Rastkerben in einem Winkelabstand von 120 ° zueinander versetzt angeordnet. Die beiden Rastmechanismen 41,42 werden von der Regeleinrichtung 40 über eine Leitung 45 betätigt.

Zur Einnahme ihrer zweiten Arbeitsstellung zueinander, d.h. bei der Betriebsart "Weißes Dauerlicht", werden die beiden Rastmechanismen 41 und 42 betätigt. Hierzu wird zunächst die Betriebsart "Weißes Dauerlicht" an der erfindungsgemäßen Vorrichtung eingestellt, und von einem Netzteil 46 der Lichterzeugungseinheit 14 wird nun der konstante Lampenstrom gemäß Figur 4 geliefert. Die Regeleinrichtung 40 steuert nun den Motor 29 mit einer geringeren Spannung an, so daß sich die beiden Filterscheiben 26,27 langsamer oder langsam drehen. Dann werden die beiden Rastmechnismen 41,42 betätigt, wobei die Rastkörper der Rastmechanismen in die Rastkerben 43, 44 einrasten. Die zweite Filterscheibe 27 wird dabei in der Stellung gemäß Figur 5 angehalten, so daß sich das weiße Farbsegment W der zweiten Filterscheibe im Strahlengang 34 der Lampe 35 befindet. Die erste Filterscheibe 26 dreht sich aus der in Figur 5 gezeigten Stellung weiter, so daß die Rastkerbe 43 nach oben gelangt und der Rastkörper des Rastmechanismus 41 in die Rastkerbe 43 eindringt und die erste Filterscheibe somit fixiert. In dieser Stellung befindet sich dann ebenfalls das weiße Farbsegment W der Filterscheibe 26 im Strahlengang 34 der Lampe 35, weil diese Filterscheibe gegenüber der zweiten Filterscheibe 27 um 120 Winkelgrade verdreht ist. Durch den dadurch bewirkten Stillstand der Antriebswelle 28 erfährt der Motor 29 eine höhere Belastung. Diese höhere Belastung wird von der Regeleinrichtung 40 erkannt, worauf der Motor 29 abgeschaltet wird. Nunmehr wird weißes Dauerlicht von der Lampe 35 bzw. vom Lichtquellengerät 6 ausgestrahlt. Es ist klar, daß hierbei die beiden Filterscheiben nicht vom Flügelrad 36 abgedeckt werden.

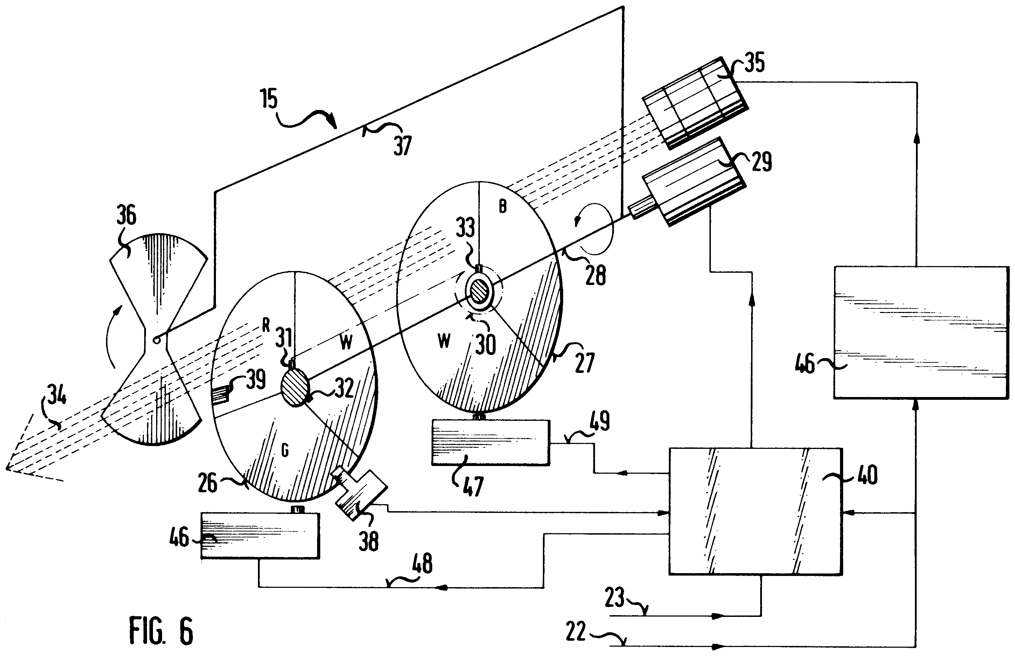

In Figur 6 ist eine abgeänderte Ausführungsform der Filtereinrichtung 15 nach Figur 5 dargestellt, wobei gleiche Bezugszeichen für gleiche Teile verwendet sind. Die wesentlichen Änderungen bestehen darin, daß anstelle der vorgenannten Rasteinrichtungen in diesem Beispiel Reibungsbremsen 46 und 47 als Fixiereinrichtung für die Filterscheiben 26 bzw. 27 vorgesehen sind. Diese Bremsen werden über Leitungen 48,49 von der Regeleinrichtung 40 gesteuert und stehen vorzugsweise mit dem Umfangsrand der Filterscheiben in Wirkverbindung. Die Verwendung von Bremsen bat den Vorteil, daß Rastkerben am Umfang der Filterscheiben entfallen, so daß die Filterscheiben bessere Gleichlaufeigenschaften aufweisen.

Im gezeigten Fall ist die Betriebsart "Sequentielles Farblicht" eingestellt, wie es sich aus der Betrachtung der Stellung der Filterscheiben 26,27 ergibt, d.h. die Farbsegmente rot, grün und blau folgen nacheinander. Für die Betriebsart "Weißes Dauerlicht" wird der entsprechende Schalter des Lichtquellengerätes 6 umgestellt, so daß über die Betriebsart-Leitung 22 ein entsprechendes Signal auf die Regeleinrichtung 40 gegeben wird. Die Regeleinrichtung 40 versorgt daraufhin den Motor 29 mit einem niedrigeren Spannungspotential, so daß sich die Antriebswelle und damit auch die Filterscheiben 26,27 langsamer drehen. Passiert die Markierung 39 der ersten Filterscheibe 26 die Lichtschranke 38 , so erhalt die Regeleinrichtung 40 einen entsprechenden Impuls und die Einrichtung 40 aktiviert daraufhin über die Leitung 49 die Bremse 47 für die zweite Filterscheibe 27, so daß diese Filterscheibe zunächst angehalten wird. Da sich die erste Filterscheibe 26, die bei diesem Beispiel einen zweiten Anschlag 32 aufweist, weiterdreht, gelangt deren zweiter Anschlag 32 unter Überwindung der Kraft der Federeinrichtung 30 gegen den Anschlag 33 der ersten Filterscheibe. Die erste Filterscheibe 26 hat sich somit gegenüber der zweiten Filterscheibe 27 um einen Winkelbereich von 120 ° entsprechend dem Winkelbereich des Farbsegmentes W der ersten Filterscheibe verdreht. Hierdurch ist das weiße Farbsegment W der ersten Filterscheibe 26 wieder achsparallel zum weißen Farbsegment W der zweiten Filterscheibe 27 ausgerichtet.

Beide Filterscheiben drehen sich nun synchron weiter, wozu die Bremse 47 so beaufschlagt wird, daß sie zwar eine Drehung der zweiten Filterscheibe 27 zuläßt, jedoch deren Stellung gegenüber der ersten Filterscheibe 26 nicht verändert. Da die Markierung 39 der ersten Filterscheibe 26 in vorbestimmtem Umfangsabstand vor dem weißen Farbsegment W der ersten Filterscheibe angeordnet und somit ein Maß für die achsparallele Ausrichtung der weißen Farbsegmente der beiden Filterscheiben ist, wird beim nächsten Passieren der Markierung 39 an der Lichtschranke 38 von dieser ein weiterer Impuls an die Regeleinrichtung 40 gegeben, die nun über die Leitung 48 die Bremse 46 für die erste Filterscheibe 26 aktiv werden läßt und diese Filterscheibe stillsetzt sowie den Motor 29 abschaltet. Die weißen Farbsegmente der beiden Filterscheiben liegen jetzt im Strahlengang 34 der Lampe 35, so daß weißes Dauerlicht am Ausgang des Lichtquellengerätes 6 zur Verfügung steht.

Wird wieder sequentielles Farblicht gewünscht, so erhalt die Regeleinrichtung 40 aufgrund der Betätigung des entsprechenden Umschalters am Lichtquellengerät 6 über die Betriebsart-Leitung 22 ein entsprechendes Signal, welches bewirkt, daß die Bremsen 46 und 47 inaktiv gemacht werden und sich daher von den Filterscheiben lösen. Die Federeinrichtung 30 wird daraufhin die zweite Filterscheibe 27 wieder so einstellen, daß deren blaues Farbsegment B wieder mit dem weißen Farbsegment W der ersten Filterscheibe in Überdeckung ist.

## Patentansprüche

1. Vorrichtung zur Lichtversorgung von Endoskopen, mit einem Lichtquellengerät, das wahlweise weißes Dauerlicht oder mittles einer mehrere Farbflächenbereiche aufweisenden Filtereinrichtung mit zwei drehantreibbaren Filterscheiben sequentielles Farblicht in das jeweilige Endoskop einspeist, dadurch gekennzeichnet, daß die eine Filterscheibe (26) mit zwei Primärfarben aufweisenden Segmenten und einem weißen Segment und die andere Filterscheibe (27) mit einem eine dritte Primärfarbe aufweisenden Segment und einem weißen Segment versehen ist und daß beide Filterscheiben (26,27) auf einer gemeinsamen Drehachse (28) rotierbar und relativ zueinander einstellbar sowie derart fixierbar angeordnet sind, daß in einer ersten Arbeitsstellung der Filterscheiben das weiße Farbsegment (W) der einen Filterscheibe (26) mit dem Farbsegment (B) der anderen Filterscheibe (27) in Überdeckung und in einer zweiten Arbeitsstellung der Filterscheiben das weiße Farbsegment (W) der einen Filterscheibe (26) mit dem weißen Farbsegment (W) der anderen Filterscheibe (27) achsparallel ausgerichtet ist.

2. Vorrichtung nach Anspruch 1, dadurch gekennzeichnet, daß die eine Filterscheibe (26) starr auf einer Antriebswelle (28) und die andere Filterscheibe (27) frei drehbar auf dieser Antriebswelle (28) angeordnet ist und daß beide Filterscheiben (26,27) mittels einer Federeinrichtung (30) miteinander gekoppelt sind.

3. Vorrichtung nach Anspruch 1, dadurch gekennzeichnet, daß die eine Filterscheibe (26) einen Anschlag (31) oder zwei voneinander beabstandete Anschläge (31,32) und die andere Filterscheibe (27) einen mit den vorgenannten Anschlägen zusammenwirkenden Anschlag (33) zum Einstellen der beiden Arbeitsstellungen der Filterscheiben aufweist.

4. Vorrichtung nach Anspruch 1, dadurch gekennzeichnet, daß für die Filterscheiben (26,27) eine Fixiereinrichtung (41,42;46,47) zum Feststellen ihrer zweiten Arbeitsstellung vorgesehen ist.

5. Vorrichtung nach Anspruch 4, dadurch gekennzeichnet, daß die Fixiereinrichtung aus einem Rastmechanismus (41,42) für jede mit einer Rastkerbe (43,44) versehene Filterscheibe besteht.

6. Vorrichtung nach Anspruch 4, dadurch gekennzeichnet, daß die Fixiereinrichtung aus einer Bremse (46,47) für jede Filtereinrichtung besteht.

7. Vorrichtung nach Anspruch 1, dadurch gekennzeichnet, daß das weiße Farbsegment (W) der einen Filterscheibe (26)einen Winkelbereich von 120 ° umfaßt, während das weiße Farbsegment (W) der anderen Filterscheibe (27) einen Winkelbereich von 240 ° umfaßt.

8. Vorrichtung nach Anspruch 1, dadurch gekennzeichnet, daß das blaue Farbsegment (B) der anderen Filterscheibe (27) einen Winkelbereich von 120 ° umfaßt.

9. Vorrichtung nach Anspruch 1, dadurch gekennzeichnet, daß für die Einstellung und den Betrieb der Filterscheiben (26,27) eine Regeleinrichtung (40) vorgesehen ist.
